# FASCICULE DE BREVET EUROPEEN

(11) **EP 2 150 205 B1**
(45) Date de publication et mention de la délivrance du brevet: **02.03.2016**
(21) Numéro de dépôt: 08805900.1
(22) Date de dépôt: 30.05.2008
(51) Int. Cl.: A61F 2/24

(54) **NÉCESSAIRE DE TRAITEMENT D'UN CONDUIT DE CIRCULATION DU SANG**
KIT ZUR VERARBEITUNG EINER BLUTKREISLAUFLEITUNG
KIT FOR PROCESSING A BLOOD CIRCULATION PIPE

(30) Priorité: 30.05.2007 FR 0755355
(43) Date de publication de la demande: 10.02.2010
(73) Titulaire: Cormove, 60540 Bornel (FR)
(72) Inventeur: STYRC, Mikolaj, L-8190 Kopstal (LU)
(74) Mandataire: Blot, Philippe Robert Emile
(86) Numéro de dépôt international: PCT/FR2008/050957
(87) Numéro de publication internationale: WO 2008/152318

(56) Documents cités:
- WO-A-2006/054107
- WO-A-2006/128185
- US-A1- 2006 178 731

## Description

La présente invention concerne un nécessaire de traitement d'un conduit de circulation du sang selon le préambule de la revendication 1.

Un tel nécessaire est destiné à être implanté en remplacement d'une valve cardiaque, comme par exemple la valve pulmonaire située à la sortie du ventricule droit. Cette valve assure une circulation univoque du flux sanguin, ce qui empêche un reflux sanguin à l'issue de la contraction ventriculaire.

Des maladies affectent les valves. En particulier; celles-ci peuvent souffrir d'une mauvaise ouverture, réduisant ainsi le flux sanguin, ou n'être que partiellement étanches, autorisant ainsi un reflux ou une régurgitation vers le ventricule ayant expulsé le flux sanguin.

Il est connu de traiter ce type de maladie de manière chirurgicale, en remplaçant la valve malade. A cet effet, il est connu d'implanter dans le passage de sortie ventriculaire, en remplacement d'une valve malade, une valve prothétique formée par une armature métallique annulaire et un obturateur souple réalisé dans un tissu d'origine animale. L'obturateur est solidarisé à demeure sur l'armature.

De telles valves présentent l'avantage de pouvoir être implantées par voie percutanée, ce qui limite les risques associés a une chirurgie lourde, notamment pour les patients âgés ou affaiblis par d'autres maladies. Le tissu d'origine animale constituant l'obturateur est généralement obtenu à partir de valves de veines jugulaires bovines prélevées sur des animaux. De telles valves sont généralement disponibles jusqu'à des diamètres environ égaux à 22 mm.

De telles valves peuvent donc être utilisées uniquement pour des patients présentant un passage de sortie ventriculaire de diamètre inférieur à 25 mm environ. Toutefois, environ 75% des patients présentent un passage dé sortie ventriculaire de diamètre supérieur à 25 mm.

Pour pallier ce problème, on connaît de l'article « Percutaneous Pulmonary Valve Replacement in a Large Right Ventricular Outflow Tract », Journal of the American College of Cardiology, Volume 43, 2004, un nécessaire du type précité. Ce nécessaire comporte un support de valve présentant une armature tubulaire présentant deux collerettes d'extrémité de grands diamètres (supérieur à 25 mm) destinés à entrer en appui avec les parois du passage de sortie ventriculaire, et un tronçon central de diamètre inférieur à 22 mm, destiné à recevoir intérieurement l'obturateur souple en tissu.

Un tel nécessaire ne donne pas entière satisfaction. En effet, pour assurer une fixation adéquate du support de valve dans le conduit, les collerettes doivent présenter une longueur importante, ce qui augmente la longueur du support de valve et rend son positionnement difficile et peu précis.

On connaît en outre de WO 2006/128185 un nécessaire de traitement d'un conduit de circulation du sang du type précité.

Un but de l'invention est donc d'obtenir un nécessaire de traitement présentant un support de valve adapté pour être implanté facilement et de manière précise dans un conduit de grand diamètre, tout en étant apte à recevoir une valve animale de faible diamètre.

A cet effet, l'invention a pour objet un nécessaire du type précité selon la partie caractérisante de la revendication 1.

Le nécessaire selon l'invention peut comprendre l'une ou plusieurs des caractéristiques des revendications 2 à 12.

L'invention sera mieux comprise à la lecture de la description qui va suivre, donnée uniquement à titre d'exemple, et faite en se référant aux dessins annexés sur lesquels :
- la Figure 1 est une vue en coupe suivant un plan médian d'un premier nécessaire selon l'invention, implanté dans un conduit de circulation du sang ;
- la Figure 2 est une vue analogue à la Figure 1, lors de l'implantation de la valve prothétique du nécessaire de la Figure 1 ;
- la Figure 3 est une vue analogue à la Figure 2 d'un deuxième nécessaire de traitement selon l'invention ;
- la Figure 4 est une vue analogue à la Figure 1 d'un troisième nécessaire de traitement selon l'invention ;
- la Figure 5 est une vue analogue à la Figure 1 d'un quatrième nécessaire de traitement selon l'invention ;
- la Figure 6 est une vue analogue à la Figure 2, lors de l'implantation du support de valve du nécessaire de la Figure 5 ; et
- la Figure 7 est une vue analogue à la Figure 1 d'un cinquième nécessaire de traitement selon l'invention, avant l'implantation d'une valve dans le support de valve.

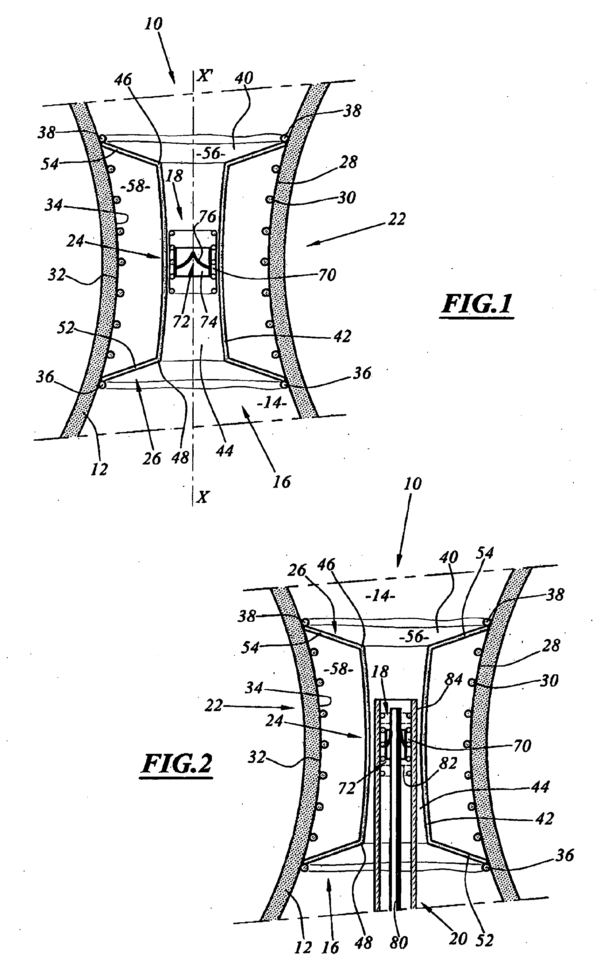

Un premier nécessaire 10 selon l'invention est représenté sur les Figures 1 et 2.

Ce nécessaire est destiné à être implanté par exemple contre une paroi intérieure 12 d'un passage de sortie ventriculaire délimitant un conduit de circulation du sang 14, en remplacement d'une valve pulmonaire native.

Le nécessaire 10 comprend un support de valve 16 destiné à être appliqué contre la paroi intérieure 12 et une valve prothétique 18 amovible portée par le support de valve 16, afin d'assurer une circulation univoque du flux sanguin dans le conduit 14.

Le nécessaire 10 comprend en outre un dispositif de largage du support 16 (non représenté) et un outil 20 de largage de la valve 18, visible sur la Figure 2.

Comme illustré par la Figure 1, le support de valve 16 comprend une paroi périphérique extérieure 22 d'appui rigide sur la surface intérieure 12, une paroi périphérique intérieure 24 disposée dans la paroi 22 pour porter la valve 18, et des moyens 26 de liaison entre la paroi intérieure 24 et la paroi extérieure 26, aptes à maintenir la paroi 24 à l'écart de la paroi extérieure 22.

La paroi extérieure 22 est formée par une armature tubulaire 28 d'axe central X-X' comprenant un treillis de fils entrelacés 30. Le treillis 30 est obtenu par tressage d'au moins un fil d'acier inoxydable, d'un alliage à mémoire de forme, ou d'un polymère. En variante, le treillis 30 est obtenu par découpe laser d'un tube.

Le treillis 30 est déployable entre un état contracté, dans lequel il présente un petit diamètre, et un état dilaté, constituant son état de repos, dans lequel il présente un grand diamètre.

Dans l'exemple représenté sur la Figure 1, le treillis 30 est déployable spontanément entre son état contracté et son état dilaté.

L'armature 28 présente une surface extérieure 32 destinée à être appliquée contre la paroi intérieure 12 du conduit 14 et une surface intérieure 34 s'étendant en regard de la paroi intérieure 24. Les surfaces 32 et 34 s'étendent entre un bord proximal 36 situé en bas sur la Figure 1, et un bord distal 38 situé en haut sur la Figure 1.

L'armature 28 définit intérieurement un passage central 40 d'axe X-X' de grand diamètre. Le passage 40 débouche dans le conduit 14 au niveau du bord proximal 36 et du bord distal 38.

Dans son état dilaté, l'armature 28 présente un diamètre supérieur à 25 mm, par exemple compris entre 25 mm et 60 mm.

La paroi périphérique intérieure 24 est formée par un tube souple 42, réalisé en tissu. Le tissu utilisé pour fabriquer le tube est par exemple du dacron, ou un autre polymère tressé ou tricoté.

Le tube souple 42 est disposé totalement dans le passage 40, de manière proximale par rapport au bord distal 38 et de manière distale par rapport au bord proximal 36. Il présente ainsi une longueur, prise suivant l'axe X-X', inférieure à la longueur de la paroi périphérique extérieure 22. Le tube 42 délimite intérieurement une lumière 44 qui débouche au niveau du bord distal 46 et du bord proximal 48 du tube 42.

La lumière 44 présente une section transversale sensiblement constante de diamètre inférieur à 35 mm, et par exemple compris entre 15 mm est 35 mm. Dans cet exemple, le rapport de la section transversale moyenne de la lumière 44 à la section transversale moyenne du passage central 40 est inférieur à 0,9.

Le tube 42 présente un diamètre extérieur inférieur au diamètre intérieur de l'armature 28. La surface extérieure du tube 42 est ainsi placée totalement à l'écart de la surface intérieure 34 dans le passage 40.

Les moyens de liaison 26 comprennent une jupe dé liaison proximale 52 et une jupe de liaison distale 54. Dans l'exemple représenté sur la Figure 1, les jupes 52, 54 sont venues de matière avec le tube 42 qu'elles prolongent à ses extrémités.

Ainsi, la jupe de liaison proximale 52 raccorde le bord proximal 48 du tube 42 au bord proximal 36 de l'armature 28 et la jupe de liaison distale 54 raccorde le bord distal 46 du tube 42 au bord distal 38 de l'armature 28. Les jupes 52, 54 s'étendent de manière périphérique autour de l'axe X-X' à partir des bords respectifs 46, 48, transversalement par rapport à l'axe X-X'.

La jupe distale 54 présente une section, prise dans un plan axial médian, qui diverge vers le bord distal 38 de l'armature 28, alors que la jupe proximale 52 présente une section qui diverge vers le bord proximal 36.

Les jupes 52, 54 et le tube 42 délimitent vers l'axe X-X' dans le passage central 40, une région intérieure 56 de circulation du sang, et, à l'écart de l'axe X-X', une région extérieure 58 annulaire.

La région intérieure 56 comprend la lumière 44 et débouche dans le conduit 14 au niveau des bords 36 et 38 de l'armature 26. La région extérieure 58 est délimitée d'une part, par les surfaces extérieures des jupes 54, 52 et du tube 42, et d'autre part, par la surface intérieure 34 de l'armature 28.

Un revêtement étanche est appliqué sur le tissu formant le tube 42 et sur les jupes 52, 54 pour isoler de manière étanche la région extérieure 58 par rapport à la région intérieure 56 et forcer le sang circulant dans le conduit 14 à passer dans la région intérieure 56 à travers la lumière 44, sans passer par la région extérieure 58.

Dans cet exemple, la valve 18 est une endovalve comprenant une endoprothèse tubulaire 70 et un obturateur souple 72 fixé dans l'endoprothèse 70. La valve 18 est mobile par rapport au support 16 lors de sa mise en place dans le support 16, entre une position initiale de stockage à l'écart du support 16 et une position montée dans le tube 42. Elle présente une longueur, prise le long de l'axe X-X' inférieure à la longueur de la paroi périphérique intérieure 24 et inférieure à la longueur de la paroi périphérique extérieure 22.

L'endoprothèse 70 est formée par un treillis tubulaire autoexpansible d'axe X-X', déployable spontanément entre une configuration rétractée radialement et une configuration dilatée radialement. Dans sa configuration dilatée, l'endoprothèse 70 est appliquée contre une surface intérieure du tube 42 dans le passage 44, comme illustré par la Figure 1, lorsque la valve occupe sa position montée dans le tube 42.

L'obturateur 72 est par exemple réalisé à base d'une valve native d'un animal bovin ou ovin. En variante, il est réalisé à base de tissu naturel ou synthétique.

L'obturateur 72 comprend une base tubulaire 74 prolongée vers le haut par trois feuillets 76 d'obturation. La base 74 est fixée sur une surface intérieure de l'endoprothèse 70.

Les feuillets 76 sont venus de matière avec la base 70 qu'ils prolongent vers le haut. Ils sont répartis autour de l'axe X-X'. Les feuillets 76 sont déformables entre une configuration d'obturation dans laquelle ils obturent sensiblement la lumière intérieure 44 et une configuration de libération, dans laquelle ils sont placés contre l'endoprothèse 70 pour permettre le passage.du sang à travers la lumière 44.

Comme connu en soi, le dispositif de largage (non représenté) du support 16 comprend un tuteur intérieur de positionnement de l'armature 28 et un fourreau extérieur de maintien de l'armature 28 dans son état contracté qui couvre l'armature 28 lors de son introduction dans le conduit 14.

L'armature 28 est ainsi insérée entre le tuteur et le fourreau dans son état contracté. Le fourreau est déplacé vers l'extrémité proximale par rapport au tuteur pour découvrir l'armature 28 et permettre le déploiement de l'armature 28.

L'outil 20 comprend de même un tuteur 80 de support de la valve 18, des fils de maintien 82 de la valve 18 sur le tuteur 80 et un cathéter extérieur 84 d'introduction de la valve 18 dans le passage 44. Un tel outil est par exemple décrit dans la demande française n°03 10298 de la Demanderesse.

La mise en place du nécessaire de traitement 10 selon l'invention dans le conduit 14 va maintenant être décrite.

Initialement, le chirurgien procède à une introduction percutanée du support de valve 16 monté sur son dispositif de largage jusqu'au passage de sortie du ventricule droit.

Lors de cette introduction, l'armature tubulaire 28 du support de valve 16 est maintenue dans son état contracté, ce qui facilite le passage dans les conduits de circulation du sang. Dans cet état, le tube en tissu 42 et les jupes de liaison 52, 54 sont comprimées radialement autour de l'axe X-X' entre le tuteur et la surface intérieure 34 de l'armature 28.

Puis, lorsque le support 16 atteint une position adéquate en regard de la paroi intérieure 12, le chirurgien procède à son largage dans le conduit 14. A cet effet, il tire proximalement le fourreau de maintien du support de valve 16 dans son état contracté pour découvrir progressivement l'armature 28 depuis son bord distal 38 vers son bord proximal 36.

Ce déplacement provoque l'expansion radiale de l'armature 28, et l'application de la surface extérieure 32 de cette armature 28 contre la paroi intérieure 12 du conduit, sur toute la longueur du support 16. Ainsi, le support 16 est fixé solidement dans le conduit 14, en minimisant son risque de déplacement, compte tenu de la surface de contact importante entre l'armature 28 et la paroi intérieure 12.

Lors du déploiement de l'armature 28 vers son état dilaté, les jupes 52, 54 se tendent entre les bords 36, 38 du support 16 et les bords 46, 48 du tube 42. Ainsi, le tube 42 s'écarte radialement de la surface intérieure 34, et se place sensiblement coaxialement avec l'axe X-X'. Dans cet état dilaté, le diamètre intérieur du passage central 40 est maximal, et est supérieur au diamètre de la lumière 44.

Puis, comme illustré par la Figure 2, la valve 18 est introduite à l'aide de son outil de largage 20 dans la lumière intérieure 44 du tube 42. Le cathéter extérieur 84 est déplacé de manière proximale par rapport au tuteur 80 pour découvrir l'endoprothèse tubulaire 70 de la valve 18. Ensuite, les fils de maintien 82 sont relâchés et retirés et l'endoprothèse tubulaire 70 se déploie depuis une configuration rétractée contre le tuteur 80 vers une configuration dilatée. Dans cette configuration dilatée, elle s'applique contre la surface intérieure du tube en tissu 42, ce qui permet sa fixation robuste.

Le diamètre de la lumière 44 étant notablement inférieur à celui du conduit 14, il est possible d'utiliser des valves natives bovines de diamètre inférieur à 35 mm pour réaliser l'obturateur 72 même avec des patients présentant un diamètre de passage de sortie ventriculaire très supérieur à 35 mm.

En fonctionnement, le sang s'écoule à travers la région intérieure 56 lorsque les feuillets de l'obturateur 72 sont plaqués contre l'endoprothèse 70 sous l'effet du flux sanguin dans un premier sens. Au contraire, les feuillets 76 obturent le passage du sang à travers la région 56 lorsque le sang circule dans un deuxième sens opposé au premier sens.

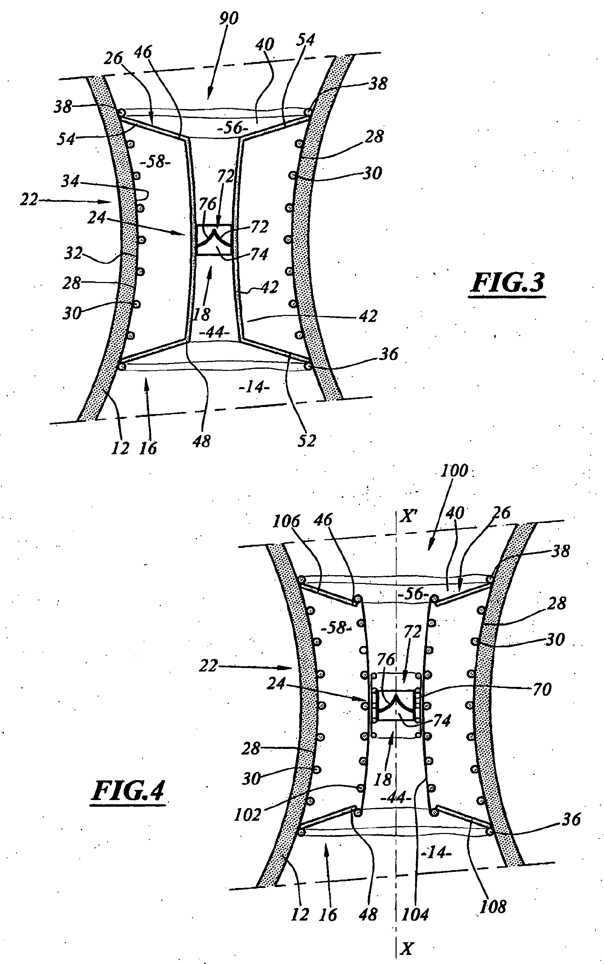

Le deuxième nécessaire de traitement 90 selon l'invention, représenté sur la Figure 3, diffère du premier nécessaire 10 en ce que la valve 18 est fixée à demeure dans le tube en tissu 42 formant la paroi périphérique intérieure 24. Ainsi, le support de valve 16 et la valve 18 sont introduits simultanément dans le conduit 14 et sont déplacés conjointement dans ce conduit.

Un troisième nécessaire de traitement 100 selon l'invention est illustré par la Figure 4. A la différence du premier nécessaire 10, la paroi périphérique intérieure 24 est formée par un treillis tubulaire 102 autoexpansible constitué d'une pluralité de fils entrelacés, noyés dans un film de polymère étanche 104.

Dans cet exemple, la longueur du treillis tubulaire 102, prise suivant l'axe X-X', est sensiblement égale à la longueur de l'armature extérieure 28. Les moyens de liaison 26 sont formés par des collerettes périphériques 106, 108 en tissu raccordant respectivement les bords distaux et les bords proximaux de l'armature 28 et du treillis tubulaire 102.

Le déploiement et le fonctionnement du troisième nécessaire 100 selon l'invention est par ailleurs analogue à celui du premier nécessaire.

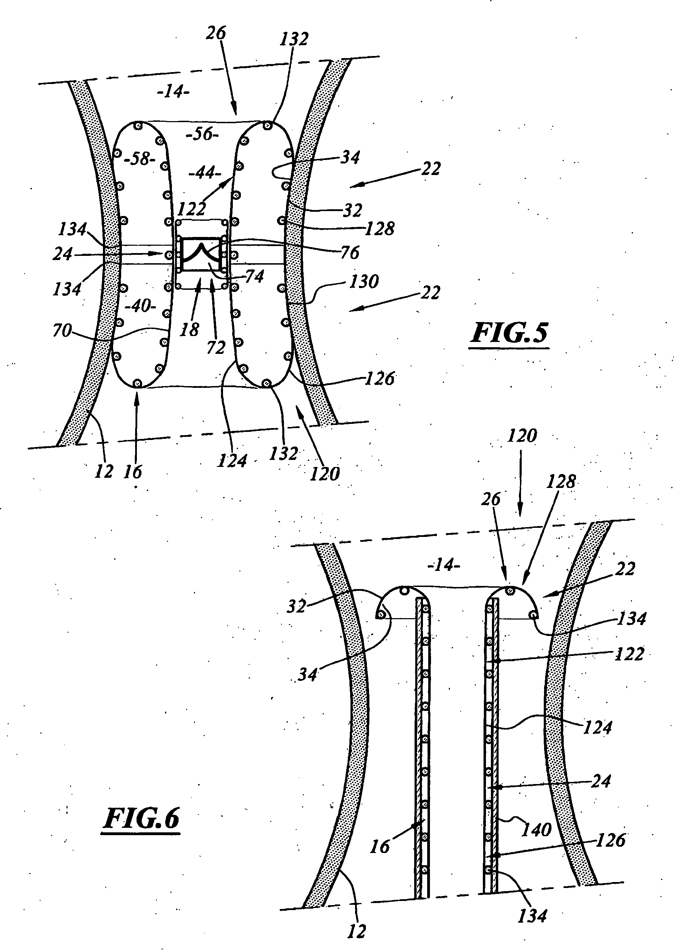

Le quatrième nécessaire selon l'invention 120 est représenté sur les Figures 5 et 6. A la différence du premier nécessaire 10, le quatrième nécessaire 120 est formé par une armature 122 tubulaire unique formée de fils entrelacés. Cette armature 122 présente un tronçon central 124 et deux tronçons d'extrémité 126, 128 retroussés extérieurement par rapport au tronçon central 124 en regard et à l'écart du tronçon central 124, autour de celui-ci.

Le tronçon central 124 forme la paroi périphérique intérieure 24 et délimite la lumière intérieure 44.

Chaque tronçon d'extrémité 126, 128 comprend une région 130 périphérique annulaire s'étendant en regard et à l'écart du tronçon central 124, et une région périphérique coudée 132 raccordant le tronçon central 124 à la région extérieure 130. Chaque région annulaire 130 forme une paroi périphérique 22 disposée en appui sur la paroi 12. Chaque région coudée 132 forme des moyens 26 de liaison entre la paroi extérieure 22 et la paroi intérieure 24.

Le tronçon d'extrémité proximale 126 et le tronçon d'extrémité distale 128 sont retroussés respectivement l'un vers l'autre. Ils délimitent chacun un passage central 40 débouchant l'un vers l'autre. Le tronçon central 124 s'étend en partie dans chacun des passages 40.

Chaque tronçon d'extrémité 126, 128 est déplaçable par pivotement de son bord libre 134 autour de la région coudée 122 entre une configuration d'introduction rétractée radialement représentée partiellement sur la Figure 6 et une configuration déployée représentée sur la Figure 5.

Dans la configuration d'introduction, les tronçons d'extrémité 126, 128 s'étendent dans le prolongement du tronçon central 124, de part et d'autre de celui-ci. Le support 16 forme alors un tube de diamètre sensiblement constant et de longueur maximale représenté partiellement sur la Figure 6. La distance entre les bords libres 134 des tronçons 126, 128 est maximale.

Dans sa configuration déployée d'utilisation, les bords libres 134 de chaque tronçon 126, 128 se sont déplacés l'un vers l'autre en regard et autour du tronçon central 124. La longueur du support 18 est alors minimale.

Avant le largage du support 16 dans le conduit 14, celui-ci est introduit et maintenu dans un cathéter 140 dans sa configuration d'introduction. Puis, le cathéter 140 est déplacé de manière proximale par rapport à l'armature 122, pour provoquer dans un premier temps le déplacement proximal du bord libre 134 du tronçon 126 qui se retrousse autour du tronçon central 124. Puis, lorsque l'armature 122 a été entièrement découverte, le bord libre 134 du tronçon d'extrémité distale 128 se déplace vers le bord libre 134 du tronçon d'extrémité distale, ce qui provoque le retroussement du tronçon d'extrémité proximale 128 autour du tronçon central 124.

Les régions extérieures 130 des tronçons 126, 128 s'appliquent alors contre la paroi intérieure 12 du conduit 14 pour maintenir en position le support 16. La valve 18 est alors introduite dans la lumière 44 comme décrit précédemment.

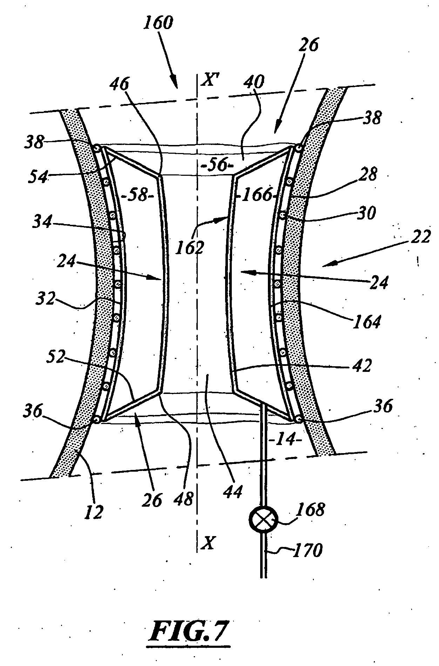

La Figure 7 illustre un cinquième nécessaire de traitement 160 selon l'invention. A la différence du premier nécessaire 10, le support de valve comprend un manchon annulaire gonflable 162 fixé sur la surface intérieure 34 de l'armature 28. Le manchon 162 est ainsi disposé dans le passage central 44.

Le manchon 162 comprend une paroi intérieure sensiblement cylindrique d'axe X-X' qui forme la paroi périphérique intérieure 24, et deux parois transversales d'extrémités qui forment les moyens de liaison 26 entre la paroi intérieure 24 et la paroi périphérique extérieure 22 sur l'armature 28.

Le manchon 162 est obturé extérieurement par une paroi périphérique étanche 164 fixée sur la surface intérieure 34.

Les parois 24, 26 et 164 délimitent entre elles un espace annuaire étanche 166 débouchant exclusivement à travers une vanne de remplissage 168 qui fait saillie axialement au voisinage du bord proximal 36 de l'armature 28.

Le manchon 162 est gonflable par introduction d'un liquide dans l'espace 166 à travers la vanne 168 entre une configuration de repos, dans laquelle le manchon est dégonflé et la paroi périphérique 24 est appliquée sur la surface intérieure et une configuration gonflée dans laquelle la paroi périphérique intérieure 24 s'étend à l'écart de la paroi périphérique extérieure 22 pour délimiter une lumière 44 de section transversale inférieure à la section transversale du passage central 40:

Lors de l'introduction du support de valve 16 dans le conduit 14, le manchon 162 est conservé dans sa configuration de repos, ce qui permet de maintenir l'armature 28 dans son état contracté de diamètre minimal.

Un cathéter 170 d'introduction d'un liquide de remplissage est par ailleurs raccordé de manière amovible à l'extrémité de la vanne 168 lors de l'introduction.

Après déploiement de l'armature 28 et application de la paroi périphérique extérieure 22 contre la surface intérieure 12, la vanne 168 est ouverte pour introduire du liquide dans l'espace annulaire 166. Le manchon 162 passe alors dans sa configuration gonflée et la valve 18 peut être introduite dans la lumière 44, comme décrit précédemment.

Après fermeture de la vanne 168, le cathéter 170 est ensuite libéré de la vanne 168 et extrait hors du conduit 14.

## Revendications

1. Nécessaire (10 ; 90 ; 100 ; 120 ; 160) de traitement d'un conduit (14) de circulation du sang, du type comprenant :
- un support de valve (16) déformable radialement entre un état contracté et un état dilaté, le support de valve (16) comprenant :
• au moins une paroi périphérique extérieure (22), destinée à s'appuyer contre une paroi (12) du conduit (14) de circulation du sang, la paroi périphérique extérieure (22) délimitant un passage central (40) d'axe central (X-X') ;
• une paroi périphérique intérieure (24), solidaire de la paroi périphérique extérieure (22), et délimitant une lumière intérieure (44) de dimension radiale inférieure à celle du passage central (40) ; et
• des moyens (26) de maintien de la paroi périphérique intérieure (24) au moins partiellement à l'écart radialement de la ou de chaque paroi périphérique extérieure (22) ; et
- une valve prothétique (18) comprenant un obturateur (72) déformable radialement, destinée à être placée sur la paroi périphérique intérieure (24) dans la lumière (44) pour obturer sélectivement la lumière (44) ;
les moyens de maintien (26) et la paroi périphérique intérieure (24) délimitant de manière étanche une région intérieure (56) de circulation du sang débouchant aux extrémités proximale et distale du support de valve (16) et contenant la lumière (44), et une région annulaire extérieure (58), pour forcer le sang circulant dans le conduit (14) à passer dans la région intérieure (56) à travers la lumière (44) ;
la paroi périphérique intérieure (24) s'étendant dans le passage central (40), axialement en regard de la ou de chaque paroi périphérique extérieure (22) ;
**caractérisé en ce que** les moyens de maintien (26) comprennent une jupe de liaison périphérique distale (54 ; 106) et une jupe de liaison périphérique proximale (52 ; 108) s'étendant dans l'espace situé entre la paroi périphérique intérieure (24) et la paroi périphérique extérieure.

2. Nécessaire (10 ; 90 ; 100 ; 160) selon la revendication 1, **caractérisé en ce que** la paroi périphérique extérieure (22) comprend une armature tubulaire (28), la paroi périphérique intérieure (24) comprenant un tube (42) totalement disposé dans le passage central (40).

3. Nécessaire selon la revendication 2, **caractérisé en ce que** le tube (42) présente une longueur, prise suivant l'axe central (X-X') inférieure à la longueur de la paroi périphérique extérieure (22).

4. Nécessaire (10 ; 90 ; 160) selon la revendication 2 ou 3, **caractérisé en ce que** l'armature (28) est formée par un treillis ajouré rigide (30), le tube (42) étant un tube souple.

5. Nécessaire (100) selon la revendication 2 ou 3, **caractérisé en ce que** l'armature (28) est formée par un treillis tubulaire extérieur ajouré (30), le tube (42) étant formé par un treillis intérieur ajouré (102) de diamètre inférieur à celui du treillis extérieur (30).

6. Nécessaire (160) selon la revendication 1, **caractérisé en ce que** le support de valve (16) comprend un manchon gonflable (162) fixé sur la paroi périphérique extérieure (22), la paroi périphérique intérieure (24) étant fixée sur ou délimitée par le manchon gonflable (162).

7. Nécessaire (120) selon la revendication 1, **caractérisé en ce que** le support de valve (16) comprend une armature tubulaire (122) unique comprenant un tronçon central tubulaire (124) formant la paroi périphérique intérieure (24) et deux tronçons d'extrémité (26, 128) retroussés à l'extérieur et autour du tronçon central (24), chaque tronçon d'extrémité (126, 128) formant une paroi périphérique extérieure (22) et des moyens de maintien (26) de la paroi périphérique intérieure (24) à l'écart de la paroi périphérique extérieure (22).

8. Nécessaire (120) selon la revendication 7, **caractérisé en ce que** les deux tronçons d'extrémité (126, 128) sont déformables spontanément entre une configuration d'introduction du support de valve (16) dans le conduit (14), dans laquelle ils s'étendent coaxialement dans le prolongement du tronçon central (124), et une configuration d'utilisation, dans laquelle ils sont retroussés autour du tronçon central (124).

9. Nécessaire (10 ; 90 ; 100 ; 120 ; 160) selon l'une quelconque des revendications précédentes, **caractérisé en ce que** la lumière centrale (44) présente une dimension transversale inférieure à 35 mm.

10. Nécessaire (10 ; 100 ; 120 ; 160) selon l'une quelconque des revendications précédentes, **caractérisé en ce que** la valve prothétique (18) est une valve déplaçable par rapport à la paroi périphérique intérieure (44), la valve déplaçable comprenant une armature porteuse (70) sur laquelle est fixé l'obturateur (72).

11. Nécessaire (90) selon l'une quelconque des revendications 1 à 8, **caractérisé en ce que** la valve prothétique (18) est fixée à demeure sur la paroi périphérique intérieure (44).

12. Nécessaire selon l'une quelconque des revendications précédentes, **caractérisé en ce que** les moyens de maintien (26) sont réalisés en tissu.

## Patentansprüche

1. Kit (10; 90; 100; 120; 160) zur Behandlung einer Blutkreislaufleitung (14) des Typs, umfassend:
- einen radial zwischen einem kontrahierten Zustand und einem erweiterten Zustand verformbaren Klappenhalter (16), wobei der Klappenhalter (16) umfasst:
• mindestens eine äußere Umfangswand (22), die bestimmt ist, sich auf einer Wand (12) der Blutkreislaufleitung (14) abzustützen, wobei die äußere Umfangswand (22) einen zentralen Durchgang (40) mit der mittigen Achse (X-X') begrenzt,
• eine innere Umfangswand (24), die mit der äußeren Umfangswand (22) fest verbunden ist und eine innere Öffnung (44) mit geringerer radialer Abmessung als die des zentralen Durchgangs (40) begrenzt, und
• Haltemittel (26) der inneren Umfangswand (24) mindestens zum Teil radial beabstandet von der oder jeder äußeren Umfangswand (22), und
- eine prothetische Klappe (18), die einen radial verformbaren Verschluss (72) umfasst, der bestimmt ist, auf der inneren Umfangswand (24) in der Öffnung (44) platziert zu sein, um die Öffnung (44) selektiv zu verschließen,
wobei die Haltemittel (26) und die innere Umfangswand (24) eine innere Blutzirkulationsregion (56), die am proximalen und distalen Ende des Klappenhalters (16) ausmündet und die Öffnung (44) enthält, und eine äußere ringförmige Region (58) dicht verschließen, um das in der Leitung (14) fließende Blut zu zwingen, in der inneren Region (56) durch die Öffnung (44) hindurchzufließen,
wobei sich die innere Umfangswand (24) in dem zentralen Durchgang (40) axial gegenüber der oder jeder äußeren Umfangswand (22) erstreckt,
**dadurch gekennzeichnet, dass** die Haltemittel (26) eine distale periphere Verbindungsschürze (54) und eine proximale periphere Verbindungsschürze (52) umfassen, die sich in dem Raum erstrecken, der sich zwischen der inneren Umfangswand (24) und der äußeren Umfangswand befindet.

2. Kit (10; 90; 100; 160) nach Anspruch 1, **dadurch gekennzeichnet, dass** die äußere Umfangswand (22) eine rohrförmige Armatur (28) umfasst, wobei die innere Umfangswand (24) ein vollständig in dem zentralen Durchgang (40) angeordnetes Rohr (42) umfasst.

3. Kit nach Anspruch 2, **dadurch gekennzeichnet, dass** das Rohr (42) eine Länge aufweist, gemessen gemäß der mittigen Achse (X-X'), die kürzer als die Länge der äußeren Umfangswand (22) ist.

4. Kit (10; 90; 160) nach Anspruch 2 oder 3, **dadurch gekennzeichnet, dass** die Armatur (28) von einem starren durchbrochenen Geflecht (30) gebildet ist, wobei das Rohr (42) ein elastisches Rohr ist.

5. Kit (100) nach Anspruch 2 oder 3, **dadurch gekennzeichnet, dass** die Armatur (28) von einem äußeren durchbrochenen rohrförmigen Geflecht (30) gebildet ist, wobei das Rohr (42) von einem inneren durchbrochenen Geflecht (102) mit einem Durchmesser, der kleiner als der des äußeren Geflechts (30) ist, gebildet ist.

6. Kit (160) nach Anspruch 1, **dadurch gekennzeichnet, dass** der Klappenhalter (16) eine aufblasbare Muffe (162) umfasst, die auf der äußeren Umfangswand (22) befestigt ist, wobei die innere Umfangswand (24) auf der aufblasbaren Muffe (162) befestigt oder von ihr begrenzt ist.

7. Kit (120) nach Anspruch 1, **dadurch gekennzeichnet, dass** der Klappenhalter (16) eine einzige rohrförmige Armatur (122) umfasst, die einen rohrförmigen zentralen Abschnitt (124), der die innere Umfangswand (24) bildet, und zwei außen und um den zentralen Abschnitt (24) umgeschlagene Endabschnitte (26, 128) umfasst, wobei jeder Endabschnitt (126, 128) eine äußere Umfangswand (22) und Haltemittel (26) der inneren Umfangswand (24) beabstandet von der äußeren Umfangswand (22) bildet.

8. Kit (120) nach Anspruch 7, **dadurch gekennzeichnet, dass** die zwei Endabschnitte (126, 128) zwischen einer Einführkonfiguration des Klappenhalters (16) in die Leitung (14), in welcher sie sich koaxial in der Verlängerung des zentralen Abschnitts (124) erstrecken, und einer Benutzungskonfiguration, in welcher sie um den zentralen Abschnitt (124) umgeschlagen sind, spontan verformbar sind.

9. Kit (10; 90; 100; 120; 160) nach einem der vorangehenden Ansprüche, **dadurch gekennzeichnet, dass** die zentrale Öffnung (44) eine transversale Abmessung unter 35 mm aufweist.

10. Kit (10; 100; 120; 160) nach einem der vorangehenden Ansprüche, **dadurch gekennzeichnet, dass** die prothetische Klappe (18) eine im Verhältnis zur inneren Umfangswand (44) verlagerbare Klappe ist, wobei die verlagerbare Klappe eine tragende Armatur (70) umfasst, auf welcher der Verschluss (72) befestigt ist.

11. Kit (90) nach einem der Ansprüche 1 bis 8, **dadurch gekennzeichnet, dass** die prothetische Klappe (18) dauerhaft auf der äußeren Umfangswand (44) befestigt ist.

12. Kit nach einem der vorangehenden Ansprüche, **dadurch gekennzeichnet, dass** die Haltemittel (26) aus Gewebe hergestellt sind.

## Claims

1. Kit (10; 90; 100; 120; 160) for treating a blood circulation vessel (14), of the type comprising:
- a valve support (16) which can be deformed radially between a contracted state and a dilated state, the valve support (16) comprising:
• at least one external peripheral wall (22) which is intended to press against a wall (12) of the blood circulation vessel (14), the external peripheral wall (22) delimiting a central passage (40) having a centre axis (X-X');
• an internal peripheral wall (24) which is fixedly joined to the external peripheral wall (22) and which delimits an internal aperture (44) having a radial dimension which is smaller than that of the central passage (40); and
• means (26) for retaining the internal peripheral wall (24) at least partially with radial spacing from the or each external peripheral wall (22); and
- a prosthetic valve (18) which comprises a shutter (72) which can be radially deformed and which is intended to be positioned on the internal peripheral wall (24) in the aperture (44) in order to selectively block the aperture (44); the retention means (26) and the internal peripheral wall (24) delimiting in a fluid-tight manner, an inner blood circulation region (56) which opens at the proximal and distal ends of the valve support (16) and which contains the aperture (44), and an external annular region (58) in order to force the blood which flows in the vessel (14) to pass into the inner region (56) through the aperture (44),
the internal peripheral wall (24) extending in the central passage (40), axially in register with the or each external peripheral wall (22), **characterised in that** the retention means (26) comprise a distal peripheral skirt (54; 106) and a proximal peripheral skirt (56; 108) which extend in the space between the internal peripheral wall (24) and the external peripheral wall..

2. Kit (10; 90; 100; 160) according to claim 1, **characterised in that** the external peripheral wall (22) comprises a tubular armature (28), the internal peripheral wall (24) comprising a tube (42) which is arranged completely inside the central passage (40).

3. Kit according to claim 2, **characterised in that** the tube (42) has a length, taken along the centre axis (X-X'), which is less than the length of the external peripheral wall (22).

4. Kit (10; 90; 160) according to claim 2 or claim 3, **characterised in that** the armature (28) is formed by a rigid open-work trellis (30), the tube (42) being a flexible tube.

5. Kit (100) according to claim 2 or claim 3, **characterised in that** the armature (28) is formed by an external tubular open-work trellis (30), the tube (42) being formed by an internal open-work trellis (102) which has a diameter smaller than that of the external trellis (30).

6. Kit (160) according to claim 1, **characterised in that** the valve support (16) comprises an inflatable sleeve (162) which is fixed to the external peripheral wall (22), the internal peripheral wall (24) being fixed to or delimited by the inflatable sleeve (162).

7. Kit (120) according to claim 1, **characterised in that** the valve support (16) comprises a single tubular armature (122) which comprises a central tubular portion (124) which forms the internal peripheral wall (24) and two end portions (26, 128) which are turned over outside and around the central portion (24), each end portion (126, 128) forming an external peripheral wall (22) and means (26) for retaining the internal peripheral wall (24) with spacing from the external peripheral wall (22).

8. Kit (120) according to claim 7, **characterised in that** the two end portions (126, 128) can be deformed spontaneously between a configuration for introducing the valve support (16) into the vessel (14), in which they extend coaxially in continuation of the central portion (124), and an operating configuration, in which they are turned over around the central portion (124).

9. Kit (10; 90; 100; 120; 160) according to any one of the preceding claims, **characterised in that** the central aperture (44) has a transverse dimension of less than 35 mm.

10. Kit (10; 100; 120; 160) according to any one of the preceding claims, **characterised in that** the prosthetic valve (18) is a valve which can be moved relative to the internal peripheral wall (44), the movable valve comprising a supporting armature (70), to which the shutter (72) is fixed.

11. Kit (90) according to any one of claims 1 to 8, **characterised in that** the prosthetic valve (18) is permanently fixed to the internal peripheral wall (44).

12. Kit according to any one of the preceding claims, **characterised in that** the retention means (26) are made of fabric.
